# EUROPEAN PATENT SPECIFICATION

(11) **EP 4 440 450 B1**
(45) Date of publication and mention of the grant of the patent: **14.10.2026**
(21) Application number: 21966029.7
(22) Date of filing: 02.12.2021
(51) Int. Cl.: A61B 17/115, A61B 90/00

(54) **SURGICAL STAPLING DEVICE WITH ANVIL TILT INDICATOR**
CHIRURGISCHE KLAMMERVORRICHTUNG MIT AMBOSSNEIGUNGSANZEIGE
DISPOSITIF D'AGRAFAGE CHIRURGICAL AVEC INDICATEUR D'INCLINAISON D'ENCLUME

(43) Date of publication of application: 09.10.2024
(73) Proprietor: Covidien LP, Mansfield, MA 02048 (US)
(72) Inventor: ZHANG, Xiliang, Shanghai 201114 (CN); WAN, Yanli, Shanghai 201114 (CN); JIAO, Yanjun, Shanghai 201114 (CN); LIU, Jinhang, Shanghai 201114 (CN); ZHANG, Zhihua, Shanghai 201114 (CN); SHEN, Xiucheng, Shanghai 201114 (CN); XU, Yishuai, Shanghai 201114 (CN)
(74) Representative: Zimmermann & Partner Patentanwälte mbB
(86) International application number: PCT/CN2021/135034
(87) International publication number: WO 2023/097597

(56) References cited:
- CN-A- 101 534 720
- CN-A- 102 186 425
- CN-A- 107 708 579
- CN-A- 108 289 683
- CN-A- 110 381 853
- US-A- 4 606 344
- US-A- 5 350 104
- US-A1- 2006 273 135
- US-A1- 2008 054 045
- US-A1- 2018 296 219
- US-A1- 2020 029 969

## Description

### FIELD

This disclosure relates to stapling devices and, more particularly, to circular stapling devices having indicators for signaling to a clinician that the stapling device has moved towards an open position a distance to allow the anvil assembly to move to a tilted position.

### BACKGROUND

Surgical stapling devices for suturing tissue are well known in the art and typically include a handle assembly, an adapter assembly, and an end effector supported on the adapter assembly for treating tissue. Such stapling devices are capable of suturing tissue more quickly than traditional suturing techniques to reduce time required to perform surgical procedures and thus reduce trauma to a patient. Typically, surgical stapling devices are designed to simultaneously cut and seal tissue.

Circular surgical stapling devices generally include an annular cartridge assembly that supports a plurality of annular rows of fasteners, and an annular anvil assembly that is operatively associated with the cartridge assembly to provide a surface against which the fasteners are formed upon a firing of the circular stapling device. The anvil assembly is movable in relation to the cartridge assembly between open and clamped positions and includes a center rod and an anvil head that is pivotably supported on a distal portion of the center rod such that the anvil head is movable from an operative position to a tilted, low-profile position. The center rod is configured to be releasably coupled to an anvil retainer of the circular stapling device to facilitate delivery of the anvil assembly to a surgical site independently of the circular stapling device. To lock the anvil head to the anvil center rod, the anvil assembly is moved from its open position towards its clamped position.

After a surgical procedure is completed, the anvil assembly needs to be moved from the clamped position towards the open position to provide spacing between the anvil head and the cartridge assembly to allow the anvil head to move to the tilted position. However, the anvil assembly should remain locked to the anvil retainer to prevent separation of the anvil assembly from the anvil retainer during removal of the circular stapling device from the surgical site.

A continuing need exists in the art for a circular stapling device that signals a clinician when the anvil assembly is in a position in which the anvil head is tilted and locked to the anvil retainer.

Documents US 2020/029969 A1, US 2018/296219 A1 and US 2008/054045 A1 disclose circular stapling devices with a clicker assembly.

### SUMMARY

The present invention is defined by appended claim 1. Specific embodiments are set forth in the dependent claims.

This disclosure is directed to a circular stapling device including a handle assembly having an indicator assembly, an elongate body, and a tool assembly that includes a reload assembly and an anvil assembly. The anvil assembly includes a center rod and an anvil head that is supported on the center rod for movement between an operative position and a tilted reduced profile position. The anvil assembly is supported on an approximation mechanism and is movable between open and clamped positions in relation to the reload assembly. The anvil assembly locks onto the approximation mechanism when the anvil assembly is located between the open and clamped positions. The indicator assembly includes a clicker assembly that provides an audible and/or tactile signal to a clinician when the anvil assembly is moved from the clamped position towards the open position a distance sufficient to allow the anvil head to pivot from the operative position to the tilted reduced profile position. The signal is produced by the indicator assembly while the anvil assembly is locked onto the approximation mechanism.

Aspects of the disclosure are directed to a circular stapling device including a handle assembly, an elongate body, a tool assembly, and an approximation member. The handle assembly includes a housing, a firing trigger, and an indicator assembly. The indicator assembly includes a clicker assembly. The elongate body has a proximal portion coupled to the handle assembly and a distal portion. The tool assembly is coupled to the distal portion of the elongate body and includes a reload assembly and an anvil assembly. The anvil assembly has a center rod and an anvil head that is coupled to the center rod for movement between an operative position and a tilted reduced profile position. The approximation mechanism includes a rotation knob, a drive screw, and an anvil retainer. The rotation knob is rotatably supported on the housing of the handle assembly and is coupled to the anvil retainer via the drive screw. The anvil retainer extends from the reload assembly and is adapted to be releasably coupled to the center rod of the anvil assembly. The approximation mechanism is operable to move the anvil assembly in relation to the reload assembly between open and clamped positions. The clicker assembly is operably associated with the approximation mechanism and is movable in response to operation of the approximation mechanism to provide an audible signal to a clinician that the anvil assembly is positioned between the open and clamped positions and spaced from the reload assembly a distance to allow movement of the anvil head from the operative position to the tilted position.

In some aspects of the disclosure, the approximation assembly includes a screw stop that is supported on the drive screw within the housing of the handle assembly.

In certain aspects of the disclosure, the screw stop is movable within the housing between advanced and retracted positions into engagement with the clicker assembly in response to operation of the approximation mechanism to provide the audible signal to a clinician.

In aspects of the disclosure, the clicker assembly includes a clicker that is movable between a proximal position and a distal position in response to movement of the screw stop from its retracted position towards its advanced position to provide the audible signal to a clinician.

In some aspects of the disclosure, the clicker includes an abutment member, and the housing of the handle assembly supports an engagement surface.

In certain aspects of the disclosure, the abutment member is movable into engagement with the engagement surface when the clicker moves from its distal position towards its proximal position to provide the audible signal to a clinician.

In aspects of the disclosure, the indicator assembly includes a biasing member that is positioned to urge the clicker assembly towards its proximal position.

In some aspects of the disclosure, the clicker assembly includes a clicker that supports a boss and the housing of the handle assembly defines a cam slot that receives the boss.

In certain aspects of the disclosure, the cam slot is configured to pivot the clicker from a first position aligned with the screw stop to a second position misaligned with the screw stop in response to movement of the screw stop from its retracted position towards its advanced position.

In aspects of the disclosure, the clicker assembly includes a restrictor that is movable from a first position spaced from the screw stop to a second position engaged with the screw stop to increase resistance to movement of the screw stop within the housing of the handle assembly.

In some aspects of the disclosure, the increased resistance provides a tactile signal to a clinician that the anvil assembly is spaced from the reload assembly a distance to allow movement of the anvil head from the operative position to the tilted position.

In certain aspects of the disclosure, the clicker defines a channel, and the restrictor is movable within the channel from the first position to the second position.

In aspects of the disclosure, the clicker assembly includes a first biasing member that is positioned to urge the restrictor towards its second position.

In some aspects of the disclosure, the clicker assembly includes a retention member that engages the restrictor to retain the restrictor in its first position.

In certain aspects of the disclosure, the clicker assembly includes a second biasing member that is positioned to urge the retention member into engagement with the restrictor.

In aspects of the disclosure, the channel includes a vertical portion and a horizontal portion, and the restrictor is received within the vertical portion and the retention member is received within the horizontal portion.

Other aspects of the disclosure are directed to a handle assembly that includes a housing, an approximation mechanism, and in indicator assembly. The housing defines a cavity and has an engagement surface. The approximation mechanism includes a rotation knob, a drive screw, and a screw stop supported within the housing on the drive screw. The screw stop is movable between advanced and retracted positions within the housing in response to operation of the rotatable member. The indicator assembly is supported within the cavity of the housing and has a clicker assembly that includes a clicker that is movable between a proximal position and a distal position. The clicker has an abutment member that is movable with the clicker from the distal position to the proximal position into engagement with the engagement surface of the housing in response to movement of the screw stop from its retracted position towards its advanced position to produce an audible signal.

Other aspects, features, and advantages will be apparent from the description and the drawings.

### BRIEF DESCRIPTION OF DRAWINGS

Various aspects of the disclosure are described herein below with reference to the drawings, wherein:
FIG. 1 is a side perspective view of a circular stapling device according to aspects of this disclosure with an anvil assembly in a clamped position;
FIG. 1A is a side perspective view of an approximation mechanism of the circular stapling device shown in FIG. 1 with the anvil assembly coupled to an anvil retainer of the approximation mechanism;
FIG. 2 is a side perspective cutaway view of a portion of a handle assembly of the circular stapling device shown in FIG. 1 with a portion of a housing of the handle assembly removed;
FIG. 3 is an exploded side perspective view of the portion of the handle assembly shown in FIG. 2;
FIG. 4 is an exploded view of an indicator assembly of the circular stapling device shown in FIG. 1;
FIG. 5 is an enlarged view of the indicated area of detail shown in FIG. 3;
FIG. 6 is a side view of the portion of the handle assembly shown in FIG. 2 with a drive screw of the approximation mechanism removed and a screw stop of the approximation mechanism in a retracted position;
FIG. 7 a side view of the portion of the handle assembly shown in FIG. 6 as the approximation mechanism is actuated to move the anvil assembly from the clamped position towards the open position and to move screw stop into engagement with a clicker of the indicator assembly;
FIG. 8 a side view of the portion of the handle assembly shown in FIG. 7 as the approximation mechanism is actuated to move the anvil assembly towards the open position and move the clicker of the indicator assembly to an actuated position;
FIG. 9 is an enlarged view of the indicated area of detail shown in FIG. 8; and
FIG. 10 is a side perspective view of a distal portion of the circular stapling device shown in FIG. 1 with the anvil assembly in a position between the open and clamped positions locked to the anvil retainer and tilted.

### DETAILED DESCRIPTION

The disclosed surgical stapling device will now be described in detail with reference to the drawings in which like reference numerals designate identical or corresponding elements in each of the several views. However, it is to be understood that the aspects of the disclosure are merely exemplary of the disclosure and may be embodied in various forms. Well-known functions or constructions are not described in detail to avoid obscuring the disclosure in unnecessary detail. Therefore, specific structural and functional details disclosed herein are not to be interpreted as limiting, but merely as a basis for the claims and as a representative basis for teaching one skilled in the art to variously employ the disclosure in virtually any appropriately detailed structure.

In this description, the term "proximal" is used generally to refer to that portion of the device that is closer to a clinician during use of the device in its customary fashion, while the term "distal" is used generally to refer to that portion of the device that is farther from the clinician during use of the device in its customary fashion. In addition, the term "clinician" is used generally to refer to medical personnel including doctors, nurses, and support personnel. Further, directional terms such as front, rear, upper, lower, top, bottom, distal, proximal, and similar terms are used to assist in understanding the description and are not intended to limit the disclosure.

A circular stapling device is disclosed that includes a handle assembly having an indicator assembly, an elongate body, and a tool assembly including an anvil assembly and a reload assembly. The anvil assembly includes a center rod and an anvil head that is supported on the center rod for pivoting movement between an operative position and a tilted, reduced profile position. The stapling device has an approximation mechanism that facilitates movement of the anvil assembly in relation to the reload assembly between open and clamped positions. The approximation mechanism includes an anvil retainer that extends from the elongate body through the reload assembly. The center rod of the anvil assembly is releasably coupled to the anvil retainer. The anvil retainer and the center rod are withdrawn into the reload assembly when the anvil assembly is moved towards the clamped position to lock the anvil assembly to the center rod. The indicator assembly is associated with the approximation mechanism. After the stapling device is fired and the anvil assembly is moving from the clamped position towards the open position, while the anvil assembly is locked to the anvil retainer, the indicator assembly provides an audible and/or a tactile signal to a clinician that the anvil head has reached a position spaced from the reload assembly a sufficient distance for the anvil head to tilt to the reduced profile position.

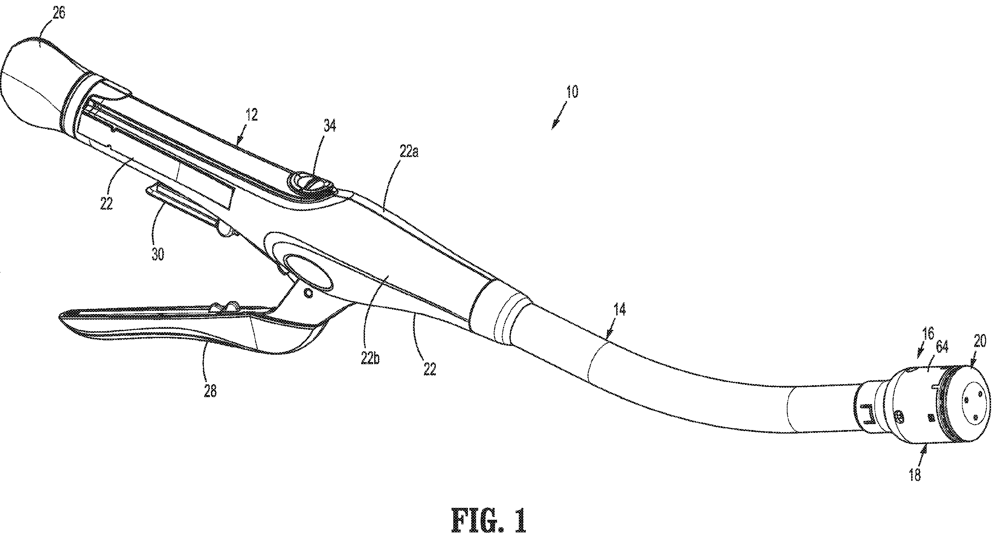

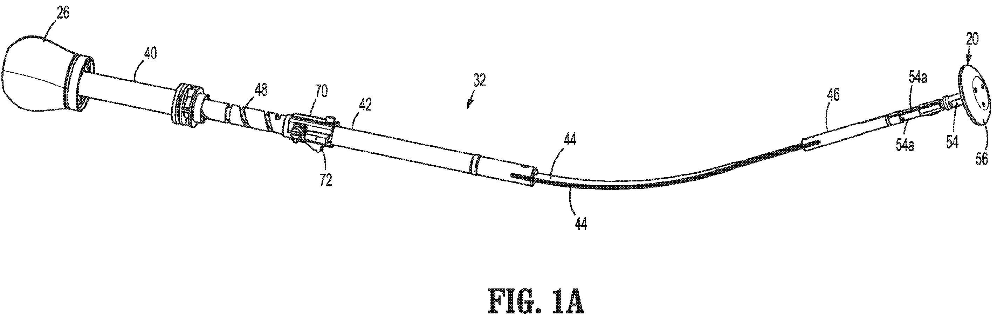

FIG. 1 illustrates a circular stapling device shown generally as stapling device 10 which includes a handle assembly 12, an elongate body 14, and a tool assembly 16. The tool assembly 16 includes a cartridge or reload assembly 18 and an anvil assembly 20. In aspects of the disclosure, the reload assembly 18 is releasably coupled to a distal portion of the elongate body 14. Alternately, the reload assembly 18 can be fixedly coupled to the distal portion of the elongate body 14.

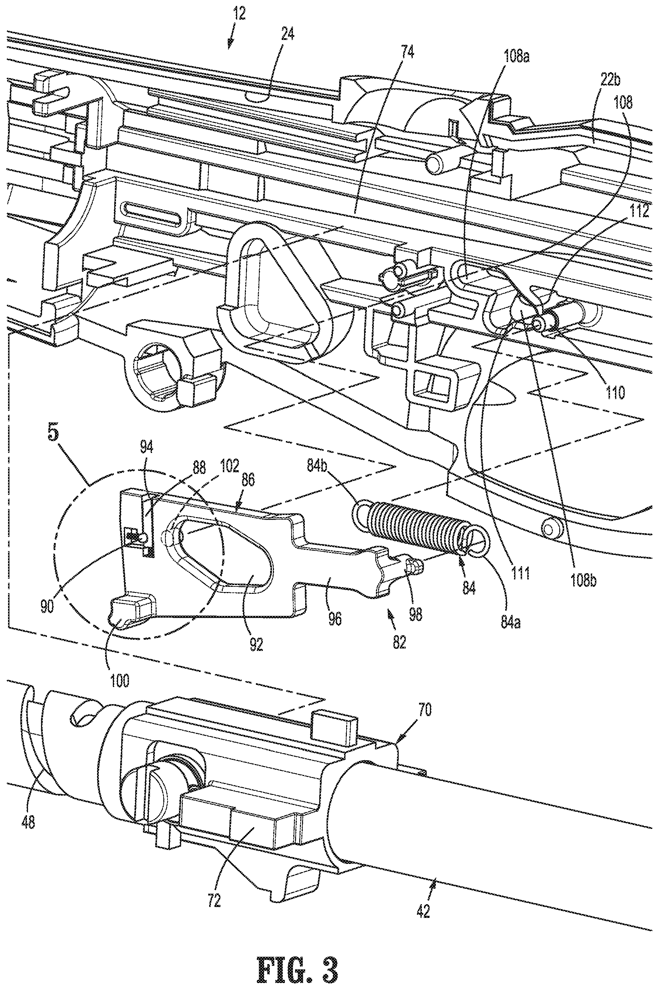

The handle assembly 12 has a grip portion or housing 22 that defines a cavity 24 (FIG. 3) that receives internal components of the handle assembly 12 that are described in further detail below. In aspects of the disclosure, the housing 22 includes half-sections 22a and 22b that are coupled together to define the cavity 24 and supports a rotation knob 26, a firing trigger 28, and a lockout member 30. The rotation knob 26 forms part of an approximation mechanism 32 (FIG. 1A) that is coupled to the anvil assembly 20 and can be actuated to move the anvil assembly 20 in relation to the reload assembly 18 between an open position and a clamped position (FIG. 1). The firing trigger 28 is supported on the housing 22 and can be actuated to eject staples from the reload assembly 18. The lockout member 30 is supported on the housing 18 and is movable from a locked position in which the lockout member 30 prevents actuation of the firing trigger 18 to an unlocked position (FIG. 1) in which the lockout member 30 permits actuation of the firing trigger 18. The handle assembly 12 also includes an indicator mechanism 34 that provides a signal to a clinician when the stapling device 10 is in a fire-ready position. For a more detailed description of the internal components of the handle assembly 12 of the stapling device 10, see, U.S. Patent No. 6,945,444 (hereinafter "the '444 Patent").

FIG. 1A illustrates the approximation mechanism 32 of the stapling device 10 (FIG. 1) which includes the rotation knob 26, a sleeve 40, a drive member, e.g., drive screw 42, one or more extensions 44, and an anvil retainer 46. The rotation knob 26 is secured to the sleeve 40 and is rotatable to cause rotation of the sleeve 40. The sleeve 40 is axially fixed to the housing of the handle assembly 12 and receives a proximal portion of the drive screw 42. The sleeve 40 supports a cam member (not shown) that extends inwardly from the sleeve 40 towards the drive screw 42. The proximal portion of the drive screw 42 defines a helical channel 48 that receives the cam member of the sleeve 40 such that rotation of the sleeve 40 causes axial movement of the drive screw 42.

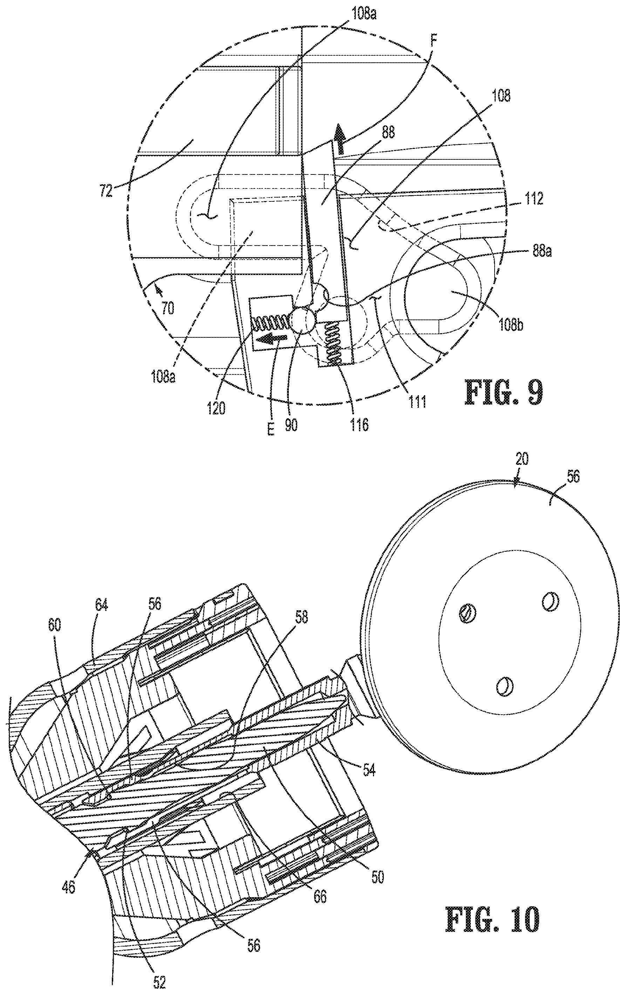

The extensions 44 have proximal portions that are secured to a distal portion of the drive screw 42 and distal portions that are secured to the anvil retainer 46. The extensions 44 extend from the drive screw 42 distally through the elongate body 14. In aspects of the disclosure, the extensions 44 include flexible bands that are supported adjacent one another and are pinned to the drive screw 42 and to the anvil retainer 46 such that axial movement of the drive screw 42 causes axial movement of the extensions 44 and the anvil retainer 46. In certain aspects of the disclosure, the anvil retainer 46 has a distal portion that defines a trocar portion 50 and a central portion that includes an annular retention member 52 (FIG. 10).

The anvil assembly 20 includes a center rod 54 and an anvil head 56. The anvil head 56 is pivotably coupled to the center rod 54 and is pivotable from an operative position (FIG. 1) to a tilted, reduced profile position (FIG. 10). The center rod 54 includes a plurality of resilient legs 54a that define a channel 58 (FIG. 10) that receives the trocar portion 50 of the anvil retainer 46 when the anvil assembly 20 is coupled to the anvil retainer 46. Each of the resilient legs 54a includes an inwardly extending engagement surface 60 (FIG. 10) that engages the annular retention member 52 of the anvil retainer 46 to couple the anvil assembly 20 to the anvil retainer 46 of the approximation mechanism 32.

The reload assembly 18 includes a housing 64 that defines a central though bore 66 (FIG. 10) that receives the anvil retainer 46 and the center rod 54 when the anvil assembly 20 is moved from the open position towards the clamped position (FIG. 1). When the resilient legs 54a of the center rod 54 are received within the central through bore 66 of the housing 64 of the reload assembly 18, the resilient legs 56 of the center rod 54 are prevented from flexing outwardly and the anvil assembly 20 is locked onto the anvil retainer 46. When the anvil assembly 20 is in the open position or near the open position spaced from the reload assembly 18, the resilient legs 54a of the center rod 54 are positioned distally of the central through bore 66 of the housing 64 of the reload assembly 18 such that the resilient legs 54a of the center rod 54 will flex outwardly upon application of a force in the distal direction on the anvil assembly 20 to release or disengage the anvil assembly 20 from the anvil retainer 46.

As described above, the anvil head 56 of the anvil assembly 20 can pivot in relation to the center rod 54 from an operative position to a tilted, reduced profile position. For a more detailed description of the operation of the tiltable anvil head of the anvil assembly 20, see the '444 Patent. The anvil head 56 is moved to the reduced profile position to allow for easier removal of the anvil assembly 20 from a body lumen. For the anvil head 56 of the anvil assembly 20 to pivot to the reduced profile position, the anvil assembly 20 must be spaced from the reload assembly 18 by a distance equal to about half the diameter of the anvil head 56 such that pivotable movement of the anvil head 56 is unobstructed by the reload assembly 18.

The approximation mechanism 32 includes a screw stop 70 (FIG. 1A) that is fixedly supported on the drive screw 42 and includes radially extending wings 72 that are received within channels 74 (FIG. 3) defined within the housing 22 of the handle assembly 12 of the stapling device 10 (FIG. 1). The screw stop 70 engages inner surfaces of the housing 64 of the handle assembly 18 in the retracted and advanced positions of the approximation mechanism 32 to define the open and clamped positions of the anvil assembly 20. For a more detailed description of the configuration and function of the screw stop 70, see the '444 Patent.

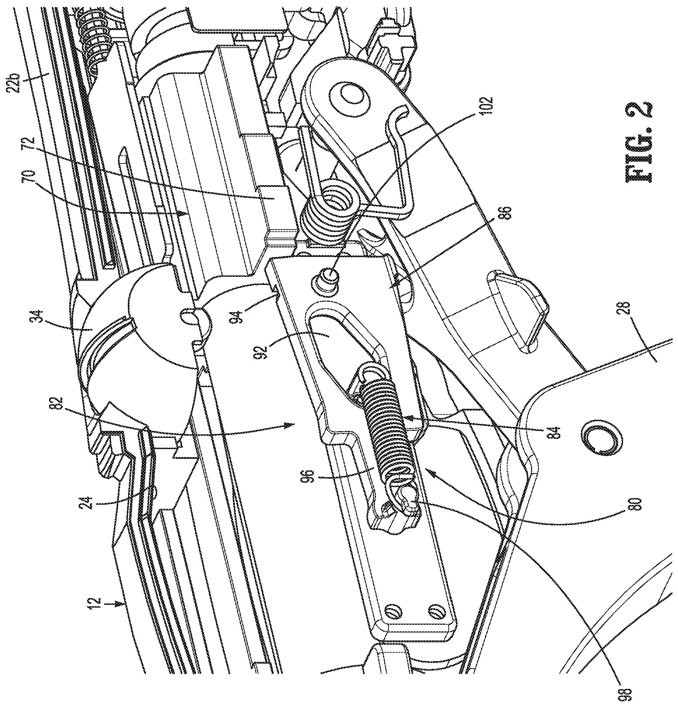

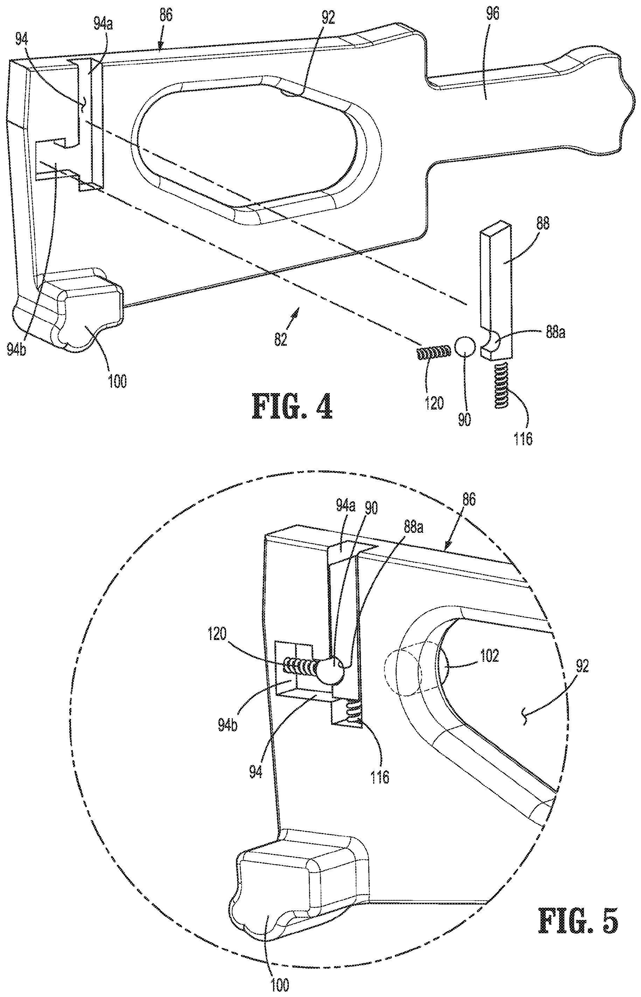

FIGS. 2-6 illustrate an indicator assembly 80 of the handle assembly 12 of the stapling device 10 (FIG. 1). The indicator assembly 80 includes a clicker assembly 82 and a biasing member 84. The clicker assembly 82 includes a clicker 86, a restrictor 88, and a retention member 90. The clicker 86 includes a body 86a that defines a central opening 92 and a channel 94 and includes a distally extending finger 96, a spring coupling member 98 (FIG. 3), an abutment member 100, and a boss 102.

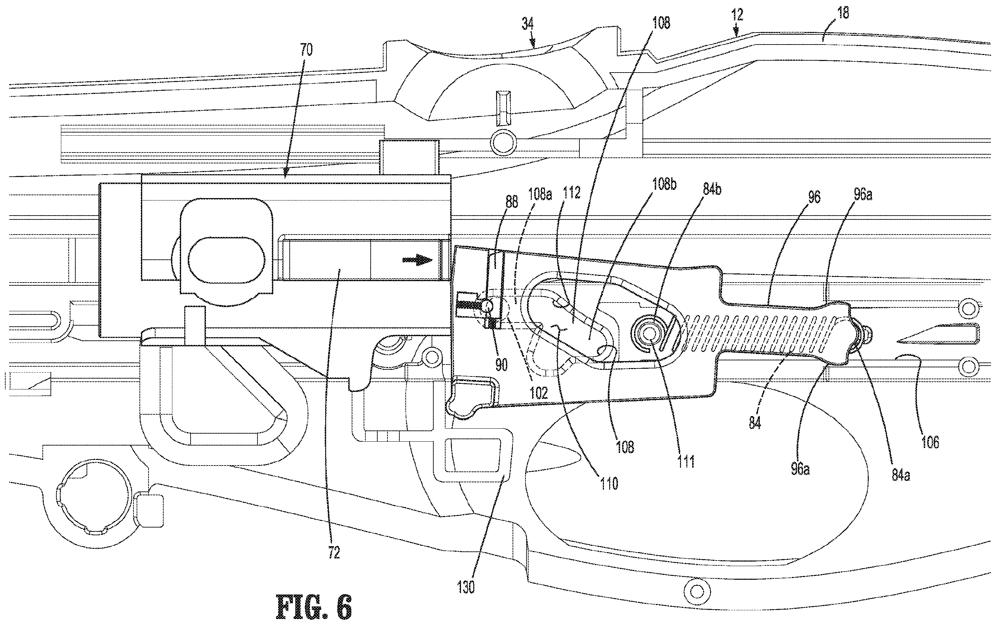

The clicker 86 is supported within the housing 18 of the handle assembly 12. More specifically, the finger 96 of the clicker 86 is received within a channel 106 (FIG. 6) of the housing 18 of the handle assembly 12 and the boss 102 is received within a cam slot 108 defined within the housing 18 of the handle assembly 12. The finger 96 has a distal portion that includes arcuate surfaces 96a (FIG. 6) that allow the distal portion of the finger 96 of the clicker 86 to pivot within the housing 18 of the handle assembly 12. The spring coupling member 98 is formed on a distal end of the finger 96 and is coupled to one end 84a of the biasing member 84. The other end 84b of the biasing member 84 is coupled to a post 110 that is secured to the housing 18 of the handle assembly 12 such that the biasing member 84 urges the clicker 86 proximally within the housing 18 of the handle assembly 12. In some aspects of the disclosure, the biasing member 84 includes a coil spring although the use of other biasing member types is envisioned.

The cam slot 108 in the housing 18 of the handle assembly 12 includes a proximal portion 108a and a distal portion 108b. The proximal portion 108a of the cam slot 108 is substantially linear and horizontal as viewed in FIG. 6, and the distal portion of the cam slot 108 defines a cavity 111 and includes a cam surface 112 that is angled downwardly from the proximal portion 108a of the cam slot 108in a distal direction.

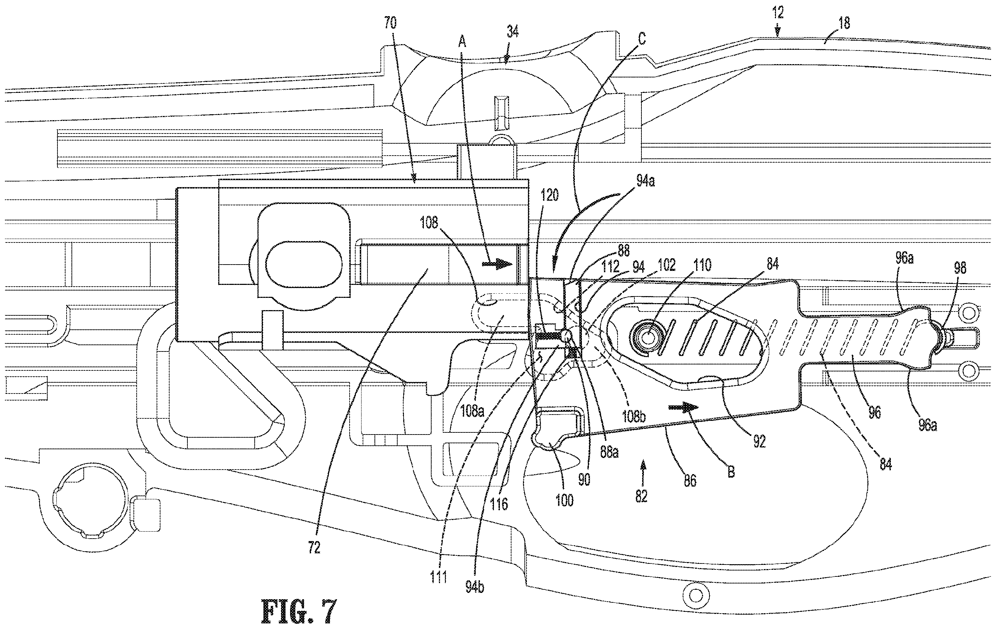

The channel 94 defined in the clicker 86 has a backward L-shaped configuration and includes a vertical portion 94a (as viewed in FIG. 7) that receives the restrictor 88 and a horizontal portion 94b that receives the retention member 90. The vertical portion 94a of the channel 94 has an open upper end. The restrictor 88 defines a concavity 88a and is movable within the vertical portion 94a from a retracted position in which the restrictor 88 is recessed within the channel 94 to an extended position in which the restrictor 88 projects from the open upper end of the channel 94. The restrictor 88 is biased to the extended position by a biasing member 116. In the extended position, the restrictor 88 is engaged with the wing 72 of the screw stop 70 to restrict movement of the screw stop 70 and, thus, movement of the drive screw 42.

The retention member 90 is received within the horizontal portion 94b of the channel 94 and is movable from a first position received within the concavity 88a of the restrictor 88 to retain the restrictor 88 in the retracted position to a second position spaced from the concavity 88a of the restrictor 88 to allow movement of the restrictor 88 from the retracted position to the extended position. In aspects of the disclosure, the retention member 90 includes a sphere and is biased towards the first position by a biasing member 120. Alternately, the retention member 90 can have a variety of configurations.

FIG. 6 illustrates the handle assembly 12 with the anvil assembly 20 in a clamped position (FIG. 1) after the stapling device 10 has been fired. In the clamped position, the drive screw 42 is in its proximal-most position and the screw stop 70 is positioned distally of the clicker 86. The clicker 86 is biased to its proximal-most position by the biasing member 84. In this position, the wing of the screw stop 70 is positioned slightly proximally of or in abutting relation to the proximal portion of the clicker 86.

FIG. 7 illustrates the handle assembly 12 as the approximation mechanism 32 (FIG. 1A) is advanced to move the anvil assembly 20 from the clamped position (FIG. 1) towards the open position. When the approximation mechanism 32 is actuated by rotating the rotation knob 26 (FIG. 1), the drive screw 42 and the screw stop 70, which is secured to the drive screw 42, move proximally within the housing 18 of the handle assembly 12 in the direction of arrow "A" such that the wing 72 of the screw stop 70 engages the proximal end of the clicker 86 of the clicker assembly 82. As the screw stop 70 moves in the direction of arrow "A", the clicker 86 of the clicker assembly 82 is pushed distally in the direction of arrow "B" against the urging of the biasing member 84. As the clicker 86 moves in the direction of arrow "B", the boss 102 on the clicker 86 engages the cam surface 112 and is pressed downwardly to pivot the clicker 86 downwardly in the direction of arrow "C".

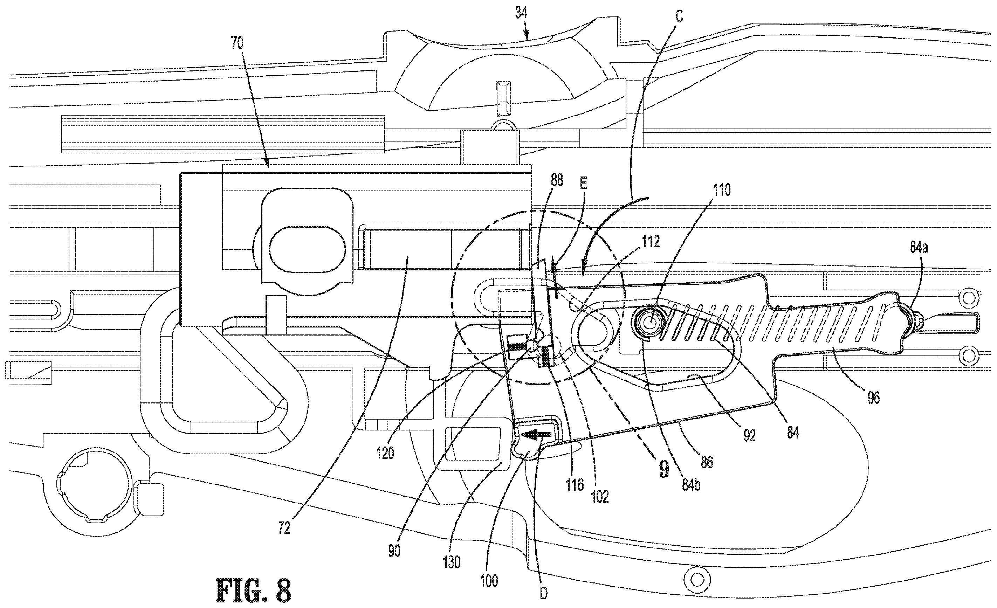

FIGS. 8-10 illustrate the handle assembly 12 as the approximation mechanism 32 (FIG. 1A) is advanced to a position in which the anvil assembly 20 is spaced from the reload assembly 18 (FIG. 1) a distance that is sufficient to allow the anvil head 56 (FIG. 10) to pivot from the operative position (FIG. 1) to the tilted reduced profile position. As the clicker 86 pivots downwardly in the direction of arrow "C", the clicker 86 moves downwardly past the wing 72 of the screw stop 70. When the clicker 86 passes below the wing 72 of the screw stop 70, the biasing member 84 snaps the clicker 86 proximally in the direction of arrow "D" such that the abutment member 100 engages an engagement surface 130 on the housing 18 of the handle assembly 12 (FIG. 1) to create an audible sound that indicates to a clinician that the anvil assembly 20 is spaced from the reload assembly 18 a distance sufficient to allow the anvil head 56 of the anvil assembly 20 to pivot to the tilted position.

When the clicker 86 moves in the direction of arrow "D" and the abutment member 100 engages the abutment surface 130 of the housing 18 of the handle assembly 12, the retention member 90 compresses the biasing member 120 and moves in the direction of arrow "E" from the concavity 88a of the restrictor 88. When this occurs, the biasing member 116 urges the restrictor 88 upwardly in the direction of arrow "F" into engagement with the wing 72 of the screw stop 70. This engagement between the restrictor 88 and the wing 72 increases resistance to movement on the screw stop 70 and on the approximation mechanism 32 that can be tactilely sensed by the clinician to further indicate to the clinician that the anvil head 56 has tilted and the stapling device 10 can be removed from the surgical site. In this position, the anvil assembly 20 is still locked onto the anvil retainer 46 and the anvil head is tilted.

Persons skilled in the art will understand that the devices and methods specifically described herein and illustrated in the accompanying drawings are non-limiting exemplary aspects of the disclosure. It is envisioned that the elements and features illustrated or described in connection with one exemplary embodiment may be combined with the elements and features of another without departing from the scope of the present disclosure. As well, one skilled in the art will appreciate further features and advantages of the disclosure based on the above-described aspects of the disclosure. Accordingly, the disclosure is not to be limited by what has been particularly shown and described, except as indicated by the appended claims.

## Claims

1. A circular stapling device (10), comprising:
a handle assembly (12) including a housing (22), a firing trigger (28), and an indicator assembly (80), the indicator assembly (80) including a clicker assembly (82);
an elongate body (14) having a proximal portion and a distal portion, the proximal portion coupled to the handle assembly (12);
a tool assembly (16) coupled to the distal portion of the elongate body (14) and including a reload assembly (18) and an anvil assembly (20), the anvil assembly (20) having a center rod (54) and an anvil head (56), the anvil head (56) coupled to the center rod (54) for movement between an operative position and a tilted reduced profile position; and
an approximation mechanism (32) including a rotation knob (26), a drive screw (42), and an anvil retainer (46), the rotation knob (26) rotatably supported on the housing (22) of the handle assembly (12) and coupled to the anvil retainer (46) via the drive screw (42), the anvil retainer (46) extending from the reload assembly (18) and adapted to be releasably coupled to the center rod (54) of the anvil assembly (20), the approximation mechanism (32) being operable to move the anvil assembly (20) in relation to the reload assembly (18) between open and clamped positions,
wherein:
the clicker assembly (82) is operably associated with the approximation mechanism (32) and movable in response to operation of the approximation mechanism to provide an audible signal to a clinician that the anvil assembly (20) is positioned between the open and clamped positions and spaced from the reload assembly (18) a distance to allow movement of the anvil head (56) from the operative position to the tilted position;
the approximation mechanism (32) includes a screw stop (70) supported on the drive screw (42) within the housing (22) of the handle assembly (12), the screw stop (70) movable within the housing (22) between proximal and distal positions in response to operation of the approximation mechanism (32) into engagement with the clicker assembly;
the clicker assembly (82) includes a clicker (86) that is movable between a proximal position and a distal position in response to movement of the screw stop (70) from its proximal position towards its distal position, wherein the clicker (86) includes an abutment member (100), and the housing (22) of the handle assembly (12) supports an engagement surface (130), the abutment member (100) movable into engagement with the engagement surface (130) to provide the audible signal to a clinician when the clicker moves (86) from its distal position towards its proximal position;
the indicator assembly (80) includes a biasing member (84) that is positioned to urge the clicker (86) towards its proximal position; and
the clicker (86) supports a boss (102) and the housing (22) of the handle assembly (12) defines a cam slot (108) that receives the boss (102), the cam slot (108) configured to pivot the clicker (86) from a first position aligned with the screw stop (70) to a second position misaligned with the screw stop (70) in response to movement of the screw stop (70) from its proximal position towards its distal position,
**characterised in that** the clicker assembly (82) further includes a restrictor (88), the restrictor (88) movable from a first position spaced from the screw stop (70) to a second position engaged with the screw stop (70) to increase resistance to movement of the screw stop (70) within the housing (22) of the handle assembly (12), the increased resistance on the screw stop (70) providing a tactile signal to a clinician that the anvil assembly (20) is spaced from the reload assembly (18) a distance to allow movement of the anvil head (56) from the operative position to the tilted position.

2. The circular stapling device of claim 1, wherein the clicker (86) defines a channel (94), and the restrictor (88) is movable within the channel (94) from the first position to the second position, and wherein the clicker assembly (82) includes a first biasing member (116) that is positioned to urge the restrictor (88) towards its second position, and further wherein the clicker assembly (82) includes a retention member (90), the retention member engaging the restrictor (88) to retain the restrictor in its first position.

3. The circular stapling device of claim 2, wherein the clicker assembly (82) includes a second biasing member (120) that is positioned to urge the retention member (90) into engagement with the restrictor (88).

4. The circular stapling device of claim 3, wherein the channel (94) includes a vertical portion (94a) and a horizontal portion (94b), the restrictor (88) received within the vertical portion (94a) of the channel and the retention member (90) received within the horizontal portion (94b) of the channel.

## Patentansprüche

1. Zirkuläre Klammervorrichtung (10) umfassend:
eine Griffanordnung (12), die ein Gehäuse (22), einen Auslöseabzug (28) und eine Anzeigeanordnung (80) aufweist, wobei die Anzeigeanordnung (80) eine Klickeranordnung (82) aufweist;
einen langgestreckten Körper (14) mit einem proximalen Abschnitt und einem distalen Abschnitt, wobei der proximale Abschnitt an die Griffanordnung (12) gekoppelt ist, und
eine Werkzeuganordnung (16), die an den distalen Abschnitt des langgestreckten Körpers (14) gekoppelt ist und eine Nachladeanordnung (18) und eine Ambossanordnung (20) aufweist, wobei die Ambossanordnung (20) eine Mittelstange (54) und einen Ambosskopf (56) aufweist, wobei der Ambosskopf (56) zur Bewegung zwischen einer Arbeitsposition und einer geneigten Position mit verringertem Profil an die Mittelstange (54) gekoppelt ist; und
einen Näherungsmechanismus (32), der einen Drehknopf (26), eine Antriebsschraube (42) und einen Ambosshalter (46) aufweist, wobei der Drehknopf (26) drehbar an dem Gehäuse (22) der Griffanordnung (12) gelagert ist und über die Antriebsschraube (42) an den Ambosshalter (46) gekoppelt ist, wobei der Ambosshalter (46) von der Nachladeanordnung (18) vorsteht und dazu ausgelegt ist, lösbar an die Mittelstange (54) der Ambossanordnung (20) gekoppelt zu werden, wobei der Annäherungsmechanismus (32) betreibbar ist, die Ambossanordnung (20) bezogen auf die Nachladeanordnung (18) zwischen einer offenen und einer geklemmten Position zu bewegen,
wobei:
die Klickeranordnung (82) mit dem Näherungsmechanismus (32) wirkverbunden ist und als Reaktion auf den Betrieb des Näherungsmechanismus beweglich ist, um einem Arzt ein hörbares Signal bereitzustellen, dass die Ambossanordnung (20) zwischen der offenen und der geklemmten Position angeordnet ist und von der Nachladeanordnung (18) um einen Abstand beabstandet ist, um Bewegung des Ambosskopfs (56) von der Arbeitsposition in die gekippte Position zu ermöglichen;
der Annäherungsmechanismus (32) einen Schraubanschlag (70) aufweist, der an der Antriebsschraube (42) innerhalb des Gehäuses (22) der Griffanordnung (12) getragen wird, wobei der Schraubanschlag (70) innerhalb des Gehäuses (22) zwischen einer proximalen und einer distalen Position als Reaktion auf den Betrieb des Annäherungsmechanismus (32) in Eingriff mit der Klickeranordnung beweglich ist;
die Klickeranordnung (82) einen Klicker (86) aufweist, der als Reaktion auf Bewegung des Schraubanschlags (70) aus seiner proximalen Position in seine distale Position zwischen einer proximalen Position und einer distalen Position beweglich ist, wobei der Klicker (86) ein Anschlagelement (100) aufweist, und das Gehäuse (22) der Griffanordnung (12) eine Eingriffsfläche (130) trägt, wobei das Anschlagelement (100) in Eingriff mit der Eingriffsfläche (130) bewegbar ist, um einem Arzt das hörbare Signal bereitzustellen, wenn sich der Klicker (86) aus seiner distalen Position in seine proximale Position bewegt;
die Indikatoranordnung (80) ein Vorspannelement (84) aufweist, das angeordnet ist, den Klicker (86) in Richtung zu seiner proximalen Position zu drücken; und
der Klicker (86) einen Vorsprung (102) trägt und das Gehäuse (22) der Griffanordnung (12) einen Nockenschlitz (108) definiert, der den Vorsprung (102) aufnimmt, wobei der Nockenschlitz (108) dazu ausgelegt ist, den Klicker (86) aus einer ersten Position, die mit dem Schraubanschlag (70) ausgerichtet ist, in eine zweite Position, die mit dem Schraubanschlag (70) fehlausgerichtet ist, zu schwenken, in Reaktion auf Bewegung des Schraubanschlags (70) aus seiner proximalen Position in Richtung zu seiner distalen Position,
**dadurch gekennzeichnet, dass** die Klickeranordnung (82) ferner einen Begrenzer (88) aufweist, wobei der Begrenzer (88) von einer ersten Position, die von dem Schraubanschlag (70) beabstandet ist, in eine zweite Position, die mit dem Schraubanschlag (70) in Eingriff steht, beweglich ist, um den Widerstand gegen Bewegung des Schraubanschlags (70) innerhalb des Gehäuses (22) der Griffanordnung (12) zu erhöhen, wobei der erhöhte Widerstand an dem Schraubanschlag (70) ein taktiles Signal an einen Arzt liefert, dass die Ambossanordnung (20) von der Nachladeanordnung (18) um einen Abstand beabstandet ist, um eine Bewegung des Ambosskopfs (56) von der Arbeitsposition in die geneigte Position zu ermöglichen.

2. Zirkuläre Klammervorrichtung nach Anspruch 1, wobei der Klicker (86) einen Kanal (94) definiert und der Begrenzer (88) innerhalb des Kanals (94) von der ersten Position in die zweite Position beweglich ist, und wobei die Klickeranordnung (82) ein erstes Vorspannelement (116) aufweist, das angeordnet ist, um den Begrenzer (88) in Richtung zu seiner zweiten Position zu drücken, und wobei ferner die Klickeranordnung (82) ein Rückhalteelement (90) aufweist, wobei das Rückhalteelement mit dem Begrenzer (88) in Eingriff steht, um den Begrenzer in seiner ersten Position zu halten.

3. Zirkuläre Klammervorrichtung nach Anspruch 2, wobei die Klammeranordnung (82) ein zweites Vorspannelement (120) aufweist, das angeordnet ist, um das Rückhalteelement (90) in Eingriff mit dem Begrenzer (88) zu drücken.

4. Zirkuläre Klammervorrichtung nach Anspruch 3, wobei der Kanal (94) einen vertikalen Abschnitt (94a) und einen horizontalen Abschnitt (94b) aufweist, wobei der Begrenzer (88) innerhalb des vertikalen Abschnitts (94a) des Kanals aufgenommen ist und das Rückhalteelement (90) innerhalb des horizontalen Abschnitts (94b) des Kanals aufgenommen ist.

## Revendications

1. Dispositif d'agrafage circulaire (10), comprenant :
un ensemble poignée (12) comprenant un logement (22), une gâchette de déclenchement (28) et un ensemble indicateur (80), l'ensemble indicateur (80) comprenant un ensemble cliquet (82) ;
un corps allongé (14) ayant une partie proximale et une partie distale, la partie proximale étant couplée à l'ensemble poignée (12) ;
un ensemble outil (16) couplé à la partie distale du corps allongé (14) et comprenant un ensemble de rechargement (18) et un ensemble enclume (20),
l'ensemble enclume (20) comportant une tige centrale (54) et une tête d'enclume (56), la tête d'enclume (56) étant couplée à la tige centrale (54) pour se déplacer entre une position opérationnelle et une position inclinée à profil réduit ; et
un mécanisme d'approximation (32) comprenant un bouton rotatif (26), une vis d'entraînement (42) et un dispositif de retenue d'enclume (46), le bouton rotatif (26) étant supporté en rotation sur le logement (22) de l'ensemble poignée (12) et couplé au dispositif de retenue d'enclume (46) par l'intermédiaire de la vis d'entraînement (42), le dispositif de retenue d'enclume (46) s'étendant depuis l'ensemble de rechargement (18) et étant adapté pour être couplé de manière amovible à la tige centrale (54) de l'ensemble enclume (20), le mécanisme d'approximation (32) étant actionnable pour déplacer l'ensemble enclume (20) par rapport à l'ensemble de rechargement (18) entre des positions ouverte et serrée,
dans lequel :
l'ensemble cliquet (82) est fonctionnellement associé au mécanisme d'approximation (32) et mobile en réponse au fonctionnement du mécanisme d'approximation pour fournir un signal audible à un clinicien indiquant que l'ensemble enclume (20) est positionné entre les positions ouverte et serrée et espacé de l'ensemble de rechargement (18) d'une distance permettant le mouvement de la tête d'enclume (56) de la position opérationnelle à la position inclinée ;
le mécanisme d'approximation (32) comprenant une butée de vis (70) supportée sur la vis d'entraînement (42) à l'intérieur du logement (22) de l'ensemble poignée (12), la butée de vis (70) pouvant se déplacer à l'intérieur du logement (22) entre des positions proximale et distale en réponse à l'actionnement du mécanisme d'approximation (32) en prise avec l'ensemble cliquet ;
l'ensemble cliquet (82) comprenant un cliquet (86) qui est mobile entre une position proximale et une position distale en réponse au mouvement de la butée de vis (70) de sa position proximale vers sa position distale, le cliquet (86) comprenant un élément de butée (100), et le logement (22) de l'ensemble poignée (12) supportant une surface de mise en prise (130), l'élément de butée (100) pouvant se déplacer en prise avec la surface de mise en prise (130) pour fournir le signal audible à un clinicien lorsque le cliquet (86) se déplace de sa position distale vers sa position proximale ;
l'ensemble indicateur (80) comprenant un élément de sollicitation (84) qui est positionné de manière à pousser le cliquet (86) vers sa position proximale ; et
le cliquet (86) supportant un bossage (102) et le logement (22) de l'ensemble poignée (12) définissant une fente de came (108) qui reçoit le bossage (102), la fente de came (108) étant configurée pour faire pivoter le cliquet (86) d'une première position alignée avec la butée de vis (70) à une seconde position désalignée avec la butée de vis (70) en réponse au mouvement de la butée de vis (70) de sa position proximale vers sa position distale,
**caractérisé en ce que** l'ensemble cliquet (82) comporte en outre un restricteur (88), le restricteur (88) étant déplaçable d'une première position espacée de la butée de vis (70) à une seconde position en prise avec la butée de vis (70) pour augmenter la résistance au déplacement de la butée de vis (70) à l'intérieur du logement (22) de l'ensemble poignée (12), la résistance accrue sur la butée de vis (70) fournissant un signal tactile à un clinicien indiquant que l'ensemble enclume (20) est espacé de l'ensemble de recharge (18) d'une distance permettant le mouvement de la tête d'enclume (56) de la position opérationnelle à la position inclinée.

2. Dispositif d'agrafage circulaire selon la revendication 1, le cliquet (86) définissant un canal (94), et le restricteur (88) étant mobile à l'intérieur du canal (94) de la première position à la seconde position, et l'ensemble cliquet (82) comprenant un premier élément de sollicitation (116) qui est positionné pour pousser le restricteur (88) vers sa seconde position, et, en outre, l'ensemble cliquet (82) comprenant un élément de retenue (90), l'élément de retenue venant en prise avec le restricteur (88) pour retenir le restricteur dans sa première position.

3. Dispositif d'agrafage circulaire selon la revendication 2, l'ensemble cliquet (82) comprenant un second élément de sollicitation (120) qui est positionné pour pousser l'élément de retenue (90) pour venir en prise avec le restricteur (88).

4. Dispositif d'agrafage circulaire selon la revendication 3, le canal (94) comprenant une partie verticale (94a) et une partie horizontale (94b), le restricteur (88) étant reçu dans la partie verticale (94a) du canal et l'élément de retenue (90) étant reçu dans la partie horizontale (94b) du canal.
